# EUROPEAN PATENT APPLICATION

(11) **EP 4 353 264 A1**
(43) Date of publication of application: **17.04.2024**
(21) Application number: 22819583.0
(22) Date of filing: 08.06.2022
(51) Int. Cl.: A61K 45/06, A61K 31/4164, A61K 31/135, A61P 33/02, A61P 33/14, A61P 27/02

(54) **USE OF CYP450 INHIBITOR IN INHIBITING OR KILLING MITES AND TREATING DRY EYE SYNDROME**

(30) Priority: 08.06.2021 CN 202110639573
(71) Applicant: Precvision Biotechnologies Ltd., Guangzhou, Guangdong 510000 (CN)
(72) Inventor: WEI, Lai, Guangzhou, Guangdong 510000 (CN)
(74) Representative: J A Kemp LLP
(86) International application number: PCT/CN2022/097684
(87) International publication number: WO 2022/257986

(57) **Abstract**

The present invention relates to a use of a CYP450 inhibitor in inhibiting or killing mites and treating xerophthalmia. A large number of experiments show that the CYP450 inhibitor can be used for inhibiting or killing mites, can effectively shorten the survival time of mites, and can be used for a mite killing and inhibiting product (such as drugs, cosmetics, daily necessities, or the like). In addition, it is found that the CYP450 inhibitor may also be used in the treatment of xerophthalmia, and can effectively alleviate and improve the symptoms of xerophthalmia.

## Description

### FIELD

The present invention relates to the field of medical technology, and in particular to a use of a CYP450 inhibitor in inhibiting or killing mites and treating xerophthalmia.

### BACKGROUND

Mites belong to a class of tiny animals of the subclass Acariformes of the class Arachnida of the phylum Arthropoda, with body sizes generally around 0.5 mm, some as small as 0.1 mm, and most species less than 1 mm. Mites have been found to have a very close relationship with the health of humans and animals (pets such as dogs and cats), such as gamasid mites, chiggers, scabies, *Demodex,* acarid mites, dust mites, and cat mites, which can suck blood, invade the skin, cause "acne rosacea" or *Demodex,* allergies, urinary tract mites, lung mites, intestinal mites, and scabies, and seriously endanger the health of humans and animals.

*Demodex* mites (belonging to Arachnid and Acarina) are microscopic ectoparasites that normally infect the pilosebaceous unit of the skin. In a wide range of reported species, at least *Demodex folliculorum* and *Demodex brevis* have been found on human body surfaces, which mainly colonize the hair follicles and sebaceous glands, meibomian glands. *Demodex* may ingest hair follicle epithelial cells, causing hair follicle expansion and hair loss, manifested as clinical blepharitis, blepharophimosis, eyelash dropout, abnormal eyelash alignment, conjunctivitis and palpebral conjunctivitis, pterygium, keratitis, eyelid basal cell carcinoma, or the like. *Demodex* may also ingest lipids, causing meibomian gland dysfunction, and leading to xerophthalmia. In addition, *Demodex* can also obstruct the meibomian gland efferent ducts by a mechanical obstruction, causing difficulty in lipid efferentation and excessive secretion retention, leading to the formation of chalazion. The clinical manifestations of eye diseases caused by *Demodex* infection include recurrent red and itchy eyes, dry eyes, burning sensation of eyes, foreign body sensation of eyes, photophobia, and increased secretion, and may be accompanied by repeated eyelash shedding; patients with severe corneal involvement may have blurred vision and decreased vision. In addition to the above eye diseases, several studies in recent years have indicated that *Demodex* infection is associated with a variety of common skin diseases, including seborrheic dermatitis, acne, rosacea, pityriasis follicularis, perioral dermatitis, *Demodex* diseases, basal cell carcinoma, or the like (see, for example, Luo, X., Li, J., Chen, C., Tseng, S. & Liang, L. Ocular Demodicosis as a Potential Cause of Ocular Surface Inflammation. Cornea 36 Suppl 1, S9-s14; Karincaoglu, Y., Tepe, B., Kalayci, B., Atambay, M. &Seyhan, M. Is Demodex folliculorum an aetiological factor in seborrhoeic dermatitis? Clinical and experimental dermatology 34, e516-520; Chen, W. &Plewig, G. Human demodicosis: revisit and a proposed classification. The British journal of dermatology 170, 1219-1225).

Mite-induced diseases (for example, eye diseases and skin diseases) have not been paid enough attention in clinical practice, and are often misdiagnosed as bacterial diseases. However, conventional antibacterial treatment often has no obvious effect.

### SUMMARY

To overcome the deficiencies of the prior art, the present invention provides a use of a CYP450 inhibitor in the preparation of products for inhibiting and/or killing mites.

Specifically, the above CYP450 is selected from one, two or more of CYP1A1, CYP1A2, CYP1B1, CYP2A6, CYP2B6, CYP2C6, CYP2C8, CYP2C9, CYP2C10, CYP2C19, CYP2D6, CYP2E1, CYP3A3, CYP3A4, CYP3A5, CYP3A7, and Pan CYP; in particular, one, two or more of CYP1A2, CYP2A6, CYP2B6, CYP2C8, CYP2C19, CYP2D6, and Pan CYP.

Specifically, the above CYP450 inhibitor may be used as the sole active ingredient or in combination with other active ingredients which may be the same or different to inhibit or kill mites.

Specifically, the above acaricidal products according to the present invention can be used for therapeutic and/or prophylactic purposes as well as for non-therapeutic and/or prophylactic purposes.

Specifically, the above mites of the present invention may be one or more of *Demodex,* dust mites, mange mites, and the like. In an embodiment of the present invention, the above mites of the present invention are *Demodex,* such as *Demodexfolliculorum* and *Demodex brevis.*

In an embodiment of the present invention, the above products for inhibiting and/or killing mites of the present invention are pharmaceutical compositions.

Specifically, the above pharmaceutical composition further includes a pharmaceutically acceptable excipient.

Specifically, the above pharmaceutical composition of the present invention can be used for the prevention and/or treatment of diseases caused by mite infection.

In an embodiment of the present invention, the above use is a use of a CYP450 inhibitor in the preparation of a drug for the prevention and/or treatment of a disease caused by mite infection.

Specifically, the above diseases may be eye diseases, skin diseases, allergic diseases, or the like.

Specifically, the above eye diseases may be one or more of blepharitis, blepharial keratoconjunctivitis, meibomian gland dysfunction, eyelash shedding, abnormal eyelash arrangement, conjunctivitis, and blepharioconjunctivitis, pterygium, keratitis, basal cell carcinoma of eyelid, xerophthalmia, chalazion, and the like; it may have one or more symptoms selected from red itching of eyes, dry eyes, burning sensation of eyes, foreign body sensation, photophobia, increased eye secretion, drop of eyelashes, blurred vision, decreased vision, and the like.

Specifically, the above skin diseases may be one or more of seborrheic dermatitis, acne, rosacea, pityriasis folliculitis, perioral dermatitis, *Demodex* diseases, scabies, basal cell carcinoma, and the like.

Specifically, the allergic diseases may be one or more of allergic asthma, allergic rhinitis, allergic dermatitis, allergic conjunctivitis, and the like.

Specifically, the pharmaceutical compositions may be in any form suitable for administration, such as topical preparations, in particular ophthalmic preparations, topical dermatological preparations, or the like.

Specifically, the above ophthalmic preparations may be eye drops, eye ointments, ophthalmic gels, ophthalmic emulsions, ophthalmic suspensions, ophthalmic films, ophthalmic solutions, intraocular injections, or the like.

Specifically, the above topical dermatological preparations may be aerosols, powders, lotions, tinctures, liniments, films, ointments, gels, pastes, emulsions, or the like.

Specifically, various dosage forms of the above pharmaceutical compositions of the present invention can be prepared according to a conventional production method in the pharmaceutical field.

Specifically, the above pharmaceutical compositions of the present invention may contain 0.01 to 99.5% (specifically, for example, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%) by weight of the active ingredient.

Specifically, the above pharmaceutical compositions can be used in humans, as well as veterinary uses in non-human animals, for example, non-human mammals, for example, pet animals (for example, dogs, cats, rabbits, mice, or the like), or livestock animals (for example, horses, cows, sheep, pigs, or the like).

In another embodiment of the present invention, the above acaricidal product of the present invention is cosmetic.

Specifically, the above cosmetics also include cosmetically acceptable excipients.

Specifically, the above cosmetic of the present invention may be a cosmetic for the face, such as a facial wash, a soap, a toner, a toner, a skincare cream, a gel, a facial cream, a sunscreen cream, an essence, a pack, a gel, a foundation, a frosting cream, or the like.

Specifically, the cosmetic of the present invention may be a cosmetic for other parts than the face, such as a neck cream, a shampoo, a body wash, a perfumed soap, a hair conditioner, a body lotion, a frosting cream, or the like.

Specifically, the above cosmetic of the present invention may also be cosmetic for eyes and periocular, such as an eye cream, a mascara, an eyeliner powder, an eyeliner cream, an eyeliner pen, an eye shadow powder, an eye shadow cream, an eyebrow pen, an eyebrow powder, or the like.

Specifically, various forms of the above cosmetics of the present invention can be prepared according to a conventional production method in the field of cosmetics.

Specifically, the cosmetic of the present invention may contain the active ingredient in an amount of 0.01 to 99.5% by weight (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%).

In another embodiment of the present invention, the above acaricidal product of the present invention is an acaricide, which can be used for killing and inhibiting mites that may colonize an article (for example, pillowcase, pillow core, bed sheet, bedding, mattress, clothing, carpet, cushion, sofa, summer sleeping mat, plush toy, air conditioner, or the like) in a living environment.

Specifically, the acaricides described above may contain any suitable excipient that can achieve the desired properties.

Specifically, the above acaricide may be in the form of a spray, a lotion, a patch, a small package, or the like.

Specifically, various forms of the above acaricide of the present invention can be prepared according to a conventional production method in the daily care product field.

Specifically, the above acaricide of the present invention may contain 0.01 to 99.5% by weight (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%) of the active ingredient.

The present invention also provides a method for the prevention and/or treatment of a disease caused by mite infection, including the step of administering to a subject in need thereof a CYP450 inhibitor or a pharmaceutical composition according to the present invention as described above.

Specifically, in the above method, the disease, the CYP450 inhibitor, and the pharmaceutical composition have the above corresponding definition of the present invention.

Specifically, the above subjects can be any animal receiving the prevention and/or treatment, especially mammals, such as humans, cats, dogs, rabbits, mice, horses, cattle, sheep, pigs, or the like.
In an embodiment of the present invention, the subject is a human; in another embodiment of the present invention, the subject is a non-human animal.

Specifically, the amount of the CYP450 inhibitor or pharmaceutical composition administered may vary depending on such factors as the route of administration, age, weight of the subject, and types and severity of the disease to be treated.

The present invention also provides a cosmetic method including the step of administering to a subject in need thereof a CYP450 inhibitor to ameliorate skin problems such as skin roughness, increased dandruff, and itching caused by mites in the subject.

Specifically, in the method, the subject is a human.

Specifically, the CYP450 inhibitor has the above respective definitions according to the present invention.

The present invention also provides a use of a CYP450 inhibitor in the preparation of drugs for preventing and/or treating xerophthalmia.

Specifically, the above CYP450 is selected from one, two or more of CYP1A1, CYP1A2, CYP1B1, CYP2A6, CYP2B6, CYP2C6, CYP2C8, CYP2C9, CYP2C10, CYP2C19, CYP2D6, CYP2E1, CYP3A3, CYP3A4, CYP3A5, CYP3A7, and Pan CYP; in particular, one, two or more of CYP1A2, CYP2A6, CYP2B6, CYP2C8, CYP2C19, CYP2D6, and Pan CYP.

Specifically, the above CYP450 inhibitor may be used as the sole active ingredient or in combination with other same or different active ingredients for the prevention and/or treatment of xerophthalmia.

Specifically, xerophthalmia has one or more symptoms selected from eye itching, foreign body sensation, burning sensation, photophobia, blurred vision, fluctuated vision, dry eyes, easy fatigue of eyes, thick secretions, sensitivity to topical stimuli, redness and swelling of eyes, congestion, keratinization, broken corneal epithelium with filamentous adhesion, and the like.

In an embodiment of the present invention, the xerophthalmia described above is xerophthalmia caused by infection with mites or related to mite infection.

Specifically, the drugs described above can be used in humans, as well as veterinary uses in non-human animals, for example, non-human mammals, for example, pet animals (for example, dogs, cats, rabbits, mice, or the like), livestock animals (for example, horses, cows, sheep, pigs, or the like).

Specifically, the above drugs may also include suitable pharmaceutically acceptable excipients.

Specifically, the drug may be in any form suitable for administration, such as an topical preparation, particularly an ophthalmic preparation, or the like.

Specifically, the above ophthalmic preparations may be eye drops, eye ointments, ophthalmic gels, ophthalmic emulsions, ophthalmic suspensions, ophthalmic films, ophthalmic solutions, intraocular injections, or the like.

The present invention also provides a method for preventing and/or treating xerophthalmia, including the step of administering to a subject in need thereof a CYP450 inhibitor or the above drug of the present invention.

Specifically, in the above method, the xerophthalmia, the CYP450 inhibitor, and the drug have the above corresponding definitions of the present invention.

Specifically, the above subjects can be any animal receiving the prevention and/or treatment, especially mammals, such as humans, cats, dogs, rabbits, mice, horses, cattle, sheep, pigs, or the like.
In an embodiment of the present invention, the subject is a human; in another embodiment of the present invention, the subject is a non-human animal.

Specifically, the amount of CYP450 inhibitor or drug administered may vary depending on such factors as the route of administration, the age and weight of the subject, and the types and severity of the disease to be treated.

The inventor discovered through a large number of experiments that the CYP450 inhibitor can be used for inhibiting or killing mites, can effectively shorten the survival time of *Demodex,* and can be used for a mite killing and inhibiting product (such as drugs, cosmetics, daily necessities, or the like). In addition, it is found that the CYP450 inhibitor may also be used in the treatment of xerophthalmia, and can effectively alleviate and improve the symptoms of xerophthalmia.

### DETAILED DESCRIPTION

Unless defined otherwise, all scientific and technical terms used herein have the same meaning as commonly understood by the ordinarily skilled in the art to which the present invention belongs.

The terms "cytochrome P450". "cytochrome P450 isoenzyme". "cytochrome P450 enzyme". and "CYP450" are used interchangeably herein and are a cytochrome b protein having iron protoporphyrin as a cofactor. CYP450 mainly includes compounds I-III; three subtypes of CYP1A1, CYP1A2, and CYP1B1 exist in CYP450 compound I, and CYP450 compound II is the largest compound in the CYP450 enzyme system, including numerous families such as CYP2A, CYP2B, CYP2C, CYP2D, CYP2E and CYP2F, for example, CYP2A6, CYP2B6, CYP2C6, CYP2C8, CYP2C9, CYP2C10, CYP2C19, CYP2D6, and CYP2E1, and CYP450 compound III mainly includes CYP3A3, CYP3A4, CYP3A5, and CYP3A7. CYP450 may be of animal, plant, and microbial, particularly, mammalian, for example, human origin.

The term "CYP450 inhibitor" refers to a substance that inhibits the activity of at least one CYP450 enzyme (as described above) by at least 1%, 5%, 10%, 20%, 30%, 40%, 50%, 55%, 60%, 65%, 70%, 75%, 80%, 85%, 90%, 95%, 96%, 97%, 98%, 99%, even 99.5%, 99.9%, or 100%.

The CYP450 inhibitor can be derived from natural products (such as effective monomers extracted from natural products, antibiotics obtained by fermentation method, semi-synthetic natural drugs, or semi-synthetic antibiotics obtained by chemical semi-synthesis or biological synthesis method using natural active substances or antibiotics as raw materials), or synthetic products (small molecule compounds obtained by chemical synthesis method), biotechnology drugs (referring to therapeutic drugs produced by DNA recombinant technology or other innovative biotechnology, such as recombinant protein or recombinant polypeptide drugs, recombinant DNA drugs, or the like).

In an embodiment of the present invention, the CYP450 inhibitor includes substances capable of inhibiting at least CYP1A1 ("CYP1A1 inhibitor"), including, but not limited to, dihydroanthraquinone, emodin, 1,4-naphthoquinone, resveratrol, methoxypsoralen, diosmetin, and pharmaceutically acceptable salts, esters, stereoisomers, derivatives, prodrugs, and solvates thereof.

In an embodiment of the present invention, the CYP450 inhibitor includes substances capable of inhibiting at least CYP1A2 ("CYP1A2 inhibitor"), including, but not limited to, acyclovir, amiodarone, atazanavir, caffeine, cimetidine, ciprofloxacin, enoxacin, famotidine, flutamide, fluvoxamine, lidocaine, lomefloxacin, mexiletine, moclobemide, norfloxacin, ofloxacin, perphenazine, propafenone, ropinirole, tacrine, ticlopidine, tocainide, verapamil, and pharmaceutically acceptable salts, esters, stereoisomers, derivatives, prodrugs, and solvates thereof.

In an embodiment of the present invention, the CYP450 inhibitor includes substances capable of inhibiting at least CYP1B1 ("CYP1B1 inhibitor"), including, but not limited to, 1,2-phenylenebis(methylene)selenocyanate, 1,3-phenylenebis(methylene)selenocyanate, 1,4-phenylenebis(methylene)selenocyanate, α-naphthoflavone, acetylenes, 2-ethynylpyrene, hesperetin, homoeriodictyol, acacetin, diosmetin, resveratrol, oltipraz, 2,4,3',5'-Tetramethoxystilbene (TMS), hydroxystilbenes, flutamide, pacilitaxel, mitoxantrone, docetaxel, tamoxifen, doxorubicin, daunomycin, trans-stilbene analogues, imperatorin, isopimpinellin, purpurin, alizarin, polycyclic aromatic hydrocarbons, apigenin, kaempferol, quercetin, amentoflavone, quercitrin, rutin, trans-resveratrol methyl ethers, 3',4'-dimethoxyflavone, 5,7,4'-trimethoxyflavone, curcumin, 7,4'-dimethoxyflavone, 7,3'-dimethoxyflavone, thiomethylstilbenes, 2,2',4,6'-Tetramethoxystilbene, methoxyflavonoids, melatonin, 2,3,4-trimethoxy-4 '-methylthio-stilbene, propargyloxyflavones, 4'-methoxy-5,7-dihydroxyflavone, 3'-fluoro-6,7,10-trimethoxy-α-naphthoflavone, coumarin, and pharmaceutically acceptable salts, esters, stereoisomers, derivatives, prodrugs, and solvates thereof.

In an embodiment of the present invention, the CYP450 inhibitor includes substances capable of inhibiting at least CYP2A6 ("CYP2A6 inhibitor"), including, but not limited to, letrozole, clotrimazole, tranylcypromine, pilocarpine, miconazole, amiodarone, ketoconazole, memantine, amphetamine, fenofibrate, methoxsalen, metyrapone, azelastine, clofibrate, fomepizole, isoniazid, menadione, nilvadipine, rosiglitazone, seratrodast, azithromycin, nicotine, triclabendazole, selegiline, and pharmaceutically acceptable salts, esters, stereoisomers, derivatives, prodrugs, and solvates thereof.

In an embodiment of the present invention, the CYP450 inhibitor includes substances capable of inhibiting at least CYP2B6 ("CYP2B6 inhibitor"), including, but not limited to, ticlopidine, orphenadrine, clotrimazole, itraconazole, raloxifene, methimazole, rilpivirine, memantine, clopidogrel, thiotepa, curcumin, sorafenib, tamoxifen, ketoconazole, crisaborole, manidipine, piperaquine, lopinavir, amprenavir, simvastatin, nelfinavir, selegiline, amlodipine, desipramine, doxorubicin, phencyclidine, azelastine, colchicine, ethanol, isoflurane, miconazole, quinidine, roxithromycin, sulfaphenazole, nitric Oxide, cisplatin, regorafenib, enzalutamide, quazepam, crizotinib, enasidenib, voriconazole, safinamide, lenvatinib, rifamycin, triclabendazole, paroxetine, pexidartinib, fluvoxamine, modafinil, curcumin sulfate, abemaciclib, elexacaftor, cenobamate, sertraline, lopinavir, ritonavir, methylene blue, abametapir, cedrol, and pharmaceutically acceptable salts, esters, stereoisomers, derivatives, prodrugs, and solvates thereof.

In an embodiment of the present invention, the CYP450 inhibitor includes substances capable of inhibiting at least CYP2C8 ("CYP2C8 inhibitor"), including, but not limited to, gemfibrozil, clopidogrel, felodipine, mometasone furoate, zafirlukast, sorafenib, erlotinib, dabrafenib, candesartan cilexetil, salmeterol, trametinib, fluticasone, fluticasone furoate, fluticasone propionate, ritonavir, clotrimazole, ketoconazole, rucaparib, pazopanib, cabozantinib, atazanavir, terbinafine, rofecoxib, quinidine, fenofibrate, bezafibrate, cimetidine, ketoprofen, pyrimethamine, ticlopidine, idelalisib, belinostat, candesartan, opicapone, tegaserod, abiraterone, ubrogepant, amoxicillin, rosiglitazone, trimethoprim, tamoxifen, irbesartan, quinine, efavirenz, rabeprazole, crisaborole, nabilone, bexarotene, nicardipine, loratadine, eltrombopag, diltiazem, enzalutamide, fluvastatin, levothyroxine, oxybutynin, medroxyprogesterone acetate, spironolactone, amlodipine, saquinavir, genistein, lenvatinib, pioglitazone, nilotinib, teriflunomide, topiroxostat, lovastatin, troglitazone, amitriptyline, cerivastatin, warfarin, lapatinib, raloxifene, quercetin, ethinylestradiol, colchicine, isoniazid, metronidazole, nilutamide, phenelzine, piroxicam, sulfaphenazole, terfenadine, triazolam, valproic acid, diethylstilbestrol, vismodegib, regorafenib, lumacaftor, midostaurin, enasidenib, letermovir, bosutinib, deferasirox, rifampicin, verapamil, simvastatin, sulfinpyrazone, montelukast, nilvadipine, compound thyroxine, mometasone, mifepristone, vemurafenib, licofelone, rutin, ponatinib, isavuconazole, rifamycin, triclabendazole, alpelisib, balaglitazone, ciglitazone, lobeglitazone, netoglitazone, rivoglitazone, tolbutamide, nifedipine, cholecalciferol, atorvastatin, losartan, mefenamic acid, troleandomycin, ezetimibe, anastrozole, abemaciclib, elexacaftor, favipiravir, methylene blue, selpercatinib, ripretinib, clofazimine, miconazole, and pharmaceutically acceptable salts, esters, stereoisomers, derivatives, prodrugs, and solvates thereof.

In an embodiment of the present invention, the CYP450 inhibitor includes substances capable of inhibiting at least CYP2C19 ("CYP2C19 inhibitor"), including, but not limited to, fluvoxamine, ticlopidine, chloramphenicol, delavirdine, gemfibrozil, stiripentol, fluoxetine, imipramine, clomipramine, lansoprazole, isoniazid, zafirlukast, tioconazole, miconazole, sertraline, efavirenz, amorphinib, eslicarbazepine acetate, abiraterone, zucapsaicin, cisapride, manidipine, artenimol, lopinavir, omeprazole, voriconazole, esomeprazole, pantoprazole, rucaparib, dovitinib, oritavancin, zonisamide, luliconazole, bortezomib, nilutamide, sitaxentan, clozapine, ethanol, nilvadipine, olanzapine, tipranavir, naloxegol, midostaurin, etoricoxib, sildenafil, citalopram, memantine, dexlansoprazole, clinafloxacin, fenofibrate, loratadine, aprepitant, ubrogepant, amiodarone, azelastine, moclobemide, nicardipine, indomethacin, progesterone, felbamate, rabeprazole, troglitazone, amitriptyline, ritonavir, mephenytoin, tranylcypromine, oxcarbazepine, ketoconazole, thalidomide, aminopyrine, fluvastatin, quinine, warfarin, diazepam, cimetidine, probenecid, carbamazepine, buprenorphine, dimethyl sulfoxide, losartan, phenelzine, sulfanilamide, telmisartan, methimazole, valproic acid, sorafenib, bicalutamide, clevidipine, vismodegib, idelalisib, topiroxostat, lobeglitazone, dosulepin, benzbromarone, enasidenib, piperaquine, isavuconazole, safinamide, lenvatinib, methsuximide, etravirine, modafinil, azelastine, amprenavir, gefitinib, seproxetine, rifamycin, fluconazole, triclabendazole, alpelisib, lynestrenol, ethinylestradiol, gestodene, paroxetine, avasimibe, curcumin sulfate, eslicarbazepine, elexacaftor, topiramate, cenobamate, atorvastatin, cyclosporine, letrozole, methylene blue, and pharmaceutically acceptable salts, esters, stereoisomers, derivatives, prodrugs, and solvates thereof.

In an embodiment of the present invention, the CYP450 inhibitor includes substances capable of inhibiting at least CYP2D6 ("CYP2D6 inhibitor"), including, but not limited to, thioridazine, paroxetine, cinacalcet, bupropion, methotrimeprazine, fluoxetine, midostaurin, propafenone, glycerol phenylbutyrate, halofantrine, cisapride, dacomitinib, orphenadrine, quinidine, fluvoxamine, venlafaxine, duloxetine, chlorpromazine, darifenacin, clozapine, celecoxib, cimetidine, tranylcypromine, chloroquine, lumefantrine, nilotinib, cholecalciferol, abiraterone, clobazam, rolapitant, panobinostat, rucaparib, manidipine, pitolisant, curcumin, delavirdine, tipranavir, vilazodone, phenylbutyric acid, sulconazole, rhein, asunaprevir, ritanserin, fusidic acid, lercanidipine, perhexiline, metoprolol, desipramine, clotrimazole, imipramine, quinine, ketoconazole, dosulepin, terfenadine, cyclosporine, sulfaphenazole, tegaserod, ritonavir, terbinafine, primaquine, nicardipine, lorcaserin, mirabegron, dronedarone, risperidone, pindolol, loratadine, nicardipine, lorcaserin, milabegron, dronedarone, risperidone, pindolol, loratadine, propranolol, imatinib, lansoprazole, mefloquine, omeprazole, selegiline, methimazole, verapamil, vinblastine, vinorelbine, temsirolimus, rabeprazole, deramciclane, peginterferon alfa-2b, entacapone, ospemifene, buprenorphine, amlodipine, cobicistat, ziprasidone, amitriptyline, vemurafenib, reboxetine, nevirapine, fluphenazine, proguanil, nefazodone, dexfenfluramine, etoricoxib, epinastine, lovastatin, saquinavir, trospium, gefitinib, lomustine, St. John's Wort, lisdexamfetamine, desvenlafaxine, amiodarone, clinafloxacin, isoniazid, escitalopram, pazopanib, asenapine, sertraline, ubrogepant, ranolazine, osilodrostat, oritavancin, lidocaine, hydroxyzine, fenfluramine, dextropropoxyphene, tamoxifen, perphenazine, promethazine, chlorpheniramine, acebutolol, moclobemide, miconazole, rotigotine, atorvastatin, cerivastatin, tripelennamine, sparteine, mibefradil, pipotiazine, biperiden, hydroxyurea, hydroxychloroquine, labetalol, mifepristone, oxprenolol, rosiglitazone, sulfonamide, sorafenib, bicalutamide, dexmedetomidine, indisulam, tapentadol, naloxegol, oxymetholone, stiripentol, levosalbutamol, vernakalant, enasidenib, artenimol, melperone, isavuconazole, black cohosh, phenelzine, safinamide, iproniazid, lenvatinib, rilpivirine, diacerein, rifamycin, dapoxetine, triclabendazole, citalopram, clemastine, clomipramine, cocaine, diphenhydramine, doxepin, doxorubicin, dexchlorpheniramine, mizolastine, trazodone, methadone, metoclopramide, ranitidine, haloperidol, amoxapine, dexchlorpheniramine maleate, ticlopidine, thiothixene, nifedipine, indinavir, oxybutynin, pimozide, azelastine, olanzapine, felodipine, pyrilamine, dimethyl sulfoxide, nicotinamide, nicotinic acid, efavirenz, nelfinavir, amodiaquine, oxamniquine, bepridil, 1-(2-Phenylethyl)-4-phenyl-4-acetoxypiperidine, cannabidiol, medical Cannabis, nabiximols, curcumin sulfate, eliglustat, everolimus, flecainide, abemaciclib, lasmiditan, elexacaftor, nortriptyline, darunavir, fedratinib, methylene blue, clofazimine, and pharmaceutically acceptable salts, esters, stereoisomers, derivatives, prodrugs, and solvates thereof.

In an embodiment of the present invention, the CYP450 inhibitor includes substances capable of at least inhibiting CYP2E1 ("CYP2E1 inhibitor"), including, but not limited to, clomethiazole, disulfiram, diethyl dithiocarbamate, isothiocyanic acid, S-adenvylmethionine, insulin, and pharmaceutically acceptable salts, esters, stereoisomers, derivatives, prodrugs, and solvates thereof.

In an embodiment of the present invention, the CYP450 inhibitor includes substances capable of inhibiting at least CYP3A4 ("CYP3A4 inhibitor"), including, but not limited to, telithromycin, clarithromycin, itraconazole, ketoconazole, indinavir, ritonavir, saquinavir, nefazodone, diltiazem, erythromycin, fluconazole, verapamil, cimetidine, amiodarone, amprenavir, aprepitant, ciprofloxacin, doxycycline, enoxacin, fluvoxamine, imatinib, miconazole, voriconazole, cedrol, and pharmaceutically acceptable salts, esters, stereoisomers, derivatives, prodrugs, and solvates thereof.

In an embodiment of the present invention, the CYP450 inhibitor includes substances capable of inhibiting at least Pan-CYP450 ("Pan-CYP450 inhibitor"), including, but not limited to, atipamezole, 1-Aminobenzotriazole (1-ABT), proadifen (SKF-525A), acetylshikonin, ketooconazole, chlorophyllin, cimitidine, satraplatin (JM216), metyrapone, miconazole, 17-octadecynoic acid, stripentol, amiodarone, and pharmaceutically acceptable salts, esters, stereoisomers thereof, derivatives, prodrugs, and solvates.

In an embodiment of the present invention, the CYP450 inhibitor also includes β-cedrene, alpha-bisabolol, chamazulene, or pogostone.

Specifically, the CYP450 inhibitor of the present invention is selected from quinidine, montelukast, quercetin, sulfaphenazole, methoxsalen, ketoconazole, itraconazole, tranylcypromine (2-PCPA), α-naphthoflavone, atipamezole, sertraline, cimetidine, curcumin, thiotepa, permethrin, clotrimazole, miconazole, paroxetine, cisapride, gemfibrozil, clopidogrel, zafirlukast, sorafenib, ritonavir, voriconazole, triclabendazole, orphenadrine, felodipine, memantine, letrozole, fluoxetine, midostaurin, metronidazole, and pharmaceutically acceptable salts, esters, stereoisomers, derivatives, prodrugs, and solvates thereof.

Specifically, the CYP450 inhibitor of the present invention is selected from quinidine, montelukast, quercetin, tranylcypromine, sertraline, thiotepa, clotrimazole, paroxetine, orphenadrine, and pharmaceutically acceptable salts, esters, stereoisomers, derivatives, prodrugs, and solvates thereof.

Specifically, the CYP450 inhibitor of the present invention is selected from methoxsalen, clopidogrel, memantine, and pharmaceutically acceptable salts, esters, stereoisomers, derivatives, prodrugs, and solvates thereof.

Specifically, the CYP450 inhibitor of the present invention is selected from cimetidine, curcumin, midostaurin, metronidazole, and pharmaceutically acceptable salts, esters, stereoisomers, derivatives, prodrugs, and solvates thereof.

In some embodiments of the present invention, the CYP450 inhibitor is selected from quinidine sulfate, montelukast Sodium, quercetin, sulfaphenazole, methoxsalen, ketoconazole, itraconazole, tranylcypromine (2-PCPA) HCl, alpha-Naphthoflavone, atipamezole, sertraline HCl, cimetidine, curcumin, thiotepa, permethrin, clotrimazole, miconazole, paroxetine, cisapride, gemfibrozil, clopidogrel Bisulfate, zafirlukast, sorafenib, ritonavir, voriconazole, triclabendazole, orphenadrine citrate, felodipine, memantine hydrochloride, letrozole, fluoxetine, midostaurin, metronidazole, cedrol, β-cedrene, alpha-bisabolol, chamazulene, and pogostone.

Specifically, the CYP450 inhibitor of the present invention is selected from quinidine sulfate, montelukast sodium, quercetin, tranylcypromine hydrochloride, sertraline hydrochloride, thiotepa, clotrimazole, paroxetine, orphenadrine citrate, methoxsalen, clopidogrel sulfate, memantine hydrochloride, cimetidine, curcumin, midostaurin, metronidazole, cedrol, β-cedrene, bisabolol, chamazulene, pogostone; more particularly, the CYP450 inhibitor of the present invention is selected from quinidine sulfate, montelukast sodium, quercetin, tranylcypromine hydrochloride, sertraline hydrochloride, thiotepa, clotrimazole, paroxetine, orphenadrine citrate, cedrol, bisabolol, chamazulene, and pogostone.

"Xerophthalmia" refers to a variety of factors caused by dry eyes as the main symptom of tear secretion disorders, often accompanied by itching, foreign body sensation, burning sensation in both eyes, photophobia, blurred vision, vision fluctuations, and other manifestations. Common symptoms include dry eyes, easy fatigue, eye itching, foreign body sensation, pain and burning sensation, thick secretions, fear of wind and light, and sensitivity to topical stimuli; sometimes the eyes are too dry and lack basic tears, instead of stimulating reflex tear secretion, resulting in frequent lacrimation; in the more serious cases, the eyes will be red and swollen, congestion, keratinization, corneal epithelium broken and filiform substance adhesion, and the long-term damage can cause corneal and conjunctival lesions and affect vision. The xerophthalmia of the present invention includes keratoconjunctivitis sicca (KCS) and further includes any one type of xerophthalmia of reduced tear secretion type and hyper-evaporative tear type. The xerophthalmia with reduced tear secretion is classified into xerophthalmia with Sjogren's syndrome and xerophthalmia without Sjogren's syndrome.

The xerophthalmia with a Sjogren's syndrome type, includes congenital anacryadenopathy, sarcoidosis, graft versus host (GVHD) disease resulting from bone marrow transplantation; accompanied by ocular pemphigus, Stevens-Johnson syndrome, trachoma, or the like; diabetes, laser (-assisted) in Situ Keratomileusis (LASIK), or the like are causes of decreased reflex secretion.

In addition, the xerophthalmia with hyper-evaporative tear type may include a condition accompanied by a decrease in the oil layer due to meibomian gland insufficiency, blepharitis, or the like; accompanied by blink insufficiency or eyelid closure insufficiency due to eyeball protrusion, rabbit eye, or the like; with decreased tear stability due to contact lens wear; accompanied by decreased mucin secretion from embryonic cells; conditions accompanied by VDT operation, or the like.

The term "prevention" or "treatment" includes therapeutic or prophylactic treatments or measures, the goal being to prevent or slow down a targeted pathological condition or disorder. A subject is successfully "prevented" or "treated" if, following administration of a therapeutically effective amount of a CYP450 inhibitor, pharmaceutical composition, or drug of the present invention according to the methods of the present invention, the subject exhibits an observable and/or measurable reduction or disappearance of one or more signs and symptoms of a particular disease. In the present invention, the term "animal" generally refers to vertebrates, particularly mammals, including humans. The term "non-human animal" refers to any vertebrate animal other than a human, particularly a mammal. In some embodiments of the present invention, the non-human animal of the present invention is a domesticated animal, that is, an animal that has been raised and domesticated by humans and whose reproduction can be controlled artificially, for functions such as eating, labor, fur, pets, experimentation, for example, commercial animals, pet animals, laboratory animals, or the like. Economic animals include such as domestic animals, for example, pigs, cattle, sheep, horses, donkeys, foxes, raccoon dogs, mink, camels, or the like. Pet animals include such as dogs, cats, rabbits, mice (for example, guinea pigs, hamsters, gerbils, chinchillas, squirrels, or the like), or the like. Experimental animals include such as monkeys, dogs, rabbits, cats, and mice (for example, rats or murine), or the like.

The disclosures of the various publications, patents, and published patent specifications cited herein are hereby incorporated by reference in their entirety.

The technical solutions in the embodiments of the present invention are clearly and completely described below with reference to FIGs. 1 to 9, and it is obvious that the described embodiments are only a part of the embodiments of the present invention, and not all of the embodiments. Based on the embodiments of the present application, all other embodiments obtained by those skilled in the art without an inventive step shall fall within the protection scope of the present application.

### Example 1: Acaricidal experiment

### 1. Experimental object

This study strictly followed the ethical principles of the Declaration of Helsinki, and Ethical Guidelines for Biomedical Research Involving Human Subjects.
In this study, *Demodex* was collected from patients diagnosed with ocular *Demodex* infection. Those meeting the inclusion criteria would be enrolled after an informed conversation and signing an informed consent form.

### 2. Demodex detection

*Demodex* detection was performed according to the conventional eyelash microscopic examination method. 3 eyelashes were extracted from each eyelid, and a total of 12 eyelashes were extracted. The extracted eyelashes were immediately placed on a glass slide, and one piece of each three eyelashes was placed under an ordinary light microscope for observation. *Demodex* at all stages were counted and classified according to morphology (specific criteria were: *Demodex brevis* at a head-to-body ratio of 1:1 and *Demodex folliculorum* at a head-to-body ratio of 1:3 to 1:4). Only adults that were fully exposed to the field of view were taken as subjects (larvae and eggs were not considered as subjects since they were more fragile in early life).

### 3. Demodex culture in vitro

Based on the above detection of *Demodex,* 50 µl of different solutions were added to each slide, and the survival of the worms was observed every 1 hour. The death was judged by observing whether the worms were active (body, limbs, or the like) under a microscope. Two skilled Demodex-related experimenters observed and judged separately during the experiment. If the judgment results were different, the third skilled experimenter was asked to make an independent judgment. The culture *in vitro* was performed in a climate chamber at 20°C and 96% humidity. Slides were transported in a humid chamber under observation and the solution was added appropriately to ensure high humidity before vacuoles appeared on solution evaporation.

### 4. Screening of anti-mite compounds

Test compounds are shown in Table 1.
(1) Compound solution preparation: Each compound was taken to prepare the solution with 0.9 % NaCl solution, with the final prepared concentration shown in Table 1;
(2) 50 µl of each concentration of the compound solution was added dropwise to the mites, and coverslips were covered;
(3) 50 µl of the solute of the compound, that is, 0.9% NaCl solution, was added dropwise to the control group, and the coverslips were covered;
(4) The number of mites and dosing time were recorded and the mites were placed in a climate chamber for cultivation;
(5) The activity of the mites was observed under a microscope regularly until the mites died;
(6) The time of death of the mites was recorded;
(7) The anti-mite activity of the compounds was statistically summarized.

The results are shown in Table 1.

**Table 1. Results of the acaricidal experiment**

| No. | Compound | Concentration | Average survival time of mites | Average survival time of mites in the control group |
|---|---|---|---|---|
| 1 | Quinidine sulfate | 10 mg/mL | 5 h | >72 h |
| | | 5 mg/mL | 5.35±2.09 h | 61.01±14.80 h |
| | | 1 mg/mL | 8.33±4.02 h | 61.01±14.80 h |
| 2 | Montelukast Sodium | 10 mg/mL | 5 h | >72 h |
| 3 | Quercetin | 10 mg/mL | 4 h | >72 h |
| 4 | Sulfaphenazole | 10 mg/mL | 70 h | >72 h |
| 5 | Methoxsalen | 10 mg/mL | 18 h | 26 h |
| 6 | Ketoconazole | 10 mg/mL | 97 h | 130 h |
| 7 | Itraconazole | 10 mg/mL | 96 h | 130 h |
| 8 | Tranylcypromine HCl | 30 mg/mL | 2.24±1.32 h | 61.01±14.80 h |
| | | 10 mg/mL | 2.89±1.08 h | 61.01±14.80 h |
| | | 3 mg/mL | 5.66±0.91 h | 61.01±14.80 h |
| | | 1 mg/mL | 7.44±6.07 h | 61.01±14.80 h |
| 9 | Sertraline HCl | 10 mg/mL | 2.50±1.37 h | ∼37.27±17.51 h |
| | | 1 mg/mL | 9.07±6.72 h | ∼37.27±17.51 h |
| 10 | Cimetidine | 10 mg/mL | 53 h | >120 h |
| 11 | Curcumin | 10 mg/mL | 45 h | >120 h |
| 12 | Thiotepa | 10 mg/mL | 22 h | >120 h |
| 13 | Clotrimazole | 3 mg/mL | ∼23.63 h | 61.01±14.80 h |
| | | 0.3 mg/mL | ∼33.01 h | 61.01±14.80 h |
| 14 | Paroxetine | 1 mg/mL | 2.44±2.04 h | 64.56±21.78 h |
| 15 | Cisapride | 10 mg/mL | 72 h | 115 h |
| 16 | Clopidogrel Bisulfate | 10 mg/mL | 23 h | 115 h |
| 17 | Voriconazole | 10 mg/mL | 72 h | >72 h |
| 18 | Orphenadrine Citrate | 10 mg/mL | 4.35±2.05 h | 45.92±23.60 h |
| | | 1 mg/mL | ∼7.09±1.44 h | 45.92±23.60 h |
| 19 | Felodipine | 10 mg/mL | 71 h | >72 h |
| 20 | Memantine HCl | 10 mg/mL | 17 h | 115 h |
| 21 | Letrozole | 10 mg/mL | 72 h | >72 h |
| 22 | Midostaurin | 10 mg/mL | 42 h | >72 h |
| 23 | Metronidazole | 10 mg/mL | 67 h | >72 h |
| 24 | Cedrol | 10 mg/mL | <20.04±1.84 h | 61.01±14.80 h |
| 25 | β-cedrene | 10 mg/mL | 42.57±8.89 h | 61.01±14.81 h |
| 26 | alpha-bisabolol | 10 mg/mL | 7.60±2.60 h | 61.01±14.82 h |
| 27 | Chamazulene | 10 mg/mL | 11.49±3.93 h | 61.01±14.83 h |
| 28 | Pogostone | 10 mg/mL | 1.90±1.06 h | 61.01±14.86 h |

### Example 2: Clinical study on treatment of dry eye caused by mite infection

### (I) Inclusion and exclusion criteria

1. Case inclusion criteria
   (1) Patients who meet the dry eye diagnostic criteria after inquiry of medical history;
   (2) 18 to 70 years old;
   (3) any gender;
   (4) Patients who can cooperate with the treatment;
2. Case exclusion criteria
   (1) Slit lamp examination for patients with iridescent cyclitis;
   (2) Patients with high or low intraocular pressure
   (3) Patients with ulceration wound on eyelid skin and corneal epithelial infiltration lesion on corneal surface
   (4) Exclusion of systemic diseases such as Sjogren's syndrome in patients with impaired liver and kidney function;
   (5) Patients under 18 years old or over 70 years old;
   (6) Those whose mental state does not allow them to cooperate in the evaluation;
   (7) Pregnant and lactating women.
3. Shedding and elimination criteria:
   (1) Patients who are unable or unwilling to continue treatment due to other diseases during treatment;
   (2) Patients who cannot cooperate or whose symptoms worsen during treatment and are unwilling to continue treatment;
   (3) Those who violate the study protocol and use other drugs that are not used in this study;
   (4) Those with incomplete final information to determine efficacy.

### (II) Endpoint indicators

Before treatment and weekly after treatment, the patient had ocular surface discomfort score, general ophthalmologic examination (visual acuity, intraocular pressure, and slit lamp examination), three tests for dry eye (conjunctival hyperemia score, BUT test, and Schirmer I test), ocular surface disease index (OSDI) score, dry eye analyzer, and ocular *Demodex* examination.

### (III) Data statistics

### 1. Sample size estimation

Considering that dry eye was a common ocular surface disease in the clinic, the positive rate of ocular *Demodex* reached 23.8% to 90.0%. Using the formula of sample size calculation for the validity test of quantitative data, it was concluded that there were 30 cases in the experimental group and 30 cases in the control group.

### 2. Statistics and analysis of study data

SPSS20.0 software and Excel were used for statistical processing. Paired sample t-test was used for normal distribution data of count data, the χ2 test was used for measurement data, and the non-parametric test was used for non-normal distribution data. The results were expressed as mean ± standard deviation (x + s), with P <0.05 as the difference.

The above is only a preferred example of the present invention and is not intended to limit the present invention, and any modifications, equivalent substitutions, or the like made within the spirit and principles of the present invention shall be included in the scope of protection of the present invention.

The foregoing examples and methods described in the present invention may vary based on the ability, experience, and preferences of the skilled in the art.

The mere listing of the steps of the method in a certain order in the present invention does not constitute any limitation on the order of the steps of the method.

## Claims

1. Use of a CYP450 inhibitor in the preparation of products for inhibiting and/or killing mites.

2. The use according to claim 1, wherein the products are pharmaceutical compositions, cosmetics, or acaricides.

3. The use according to claim 2, wherein the pharmaceutical compositions are pharmaceutical compositions for the prevention and/or treatment of diseases caused by mite infection; and
the diseases are selected from eye diseases, skin diseases, and allergic diseases;
preferably, the eye diseases are selected from blepharitis, palpebral limbic keratoconjunctivitis, meibomian gland dysfunction, eyelash loss, abnormal eyelash arrangement, conjunctivitis, and palpebral conjunctivitis, pterygium, keratitis, eyelid basal cell carcinoma, dry eye, and chalazion;
preferably, the skin diseases are selected from seborrheic dermatitis, acne, rosacea, pityriasis folliculitis, perioral dermatitis, *Demodex* diseases, sarcoptic mange, and basal cell carcinoma; and
preferably, the allergic diseases are selected from allergic asthma, allergic rhinitis, allergic dermatitis, and allergic conjunctivitis.

4. The use according to claim 2, wherein the pharmaceutical compositions are topical preparations and preferably ophthalmic preparations or topical dermatological preparations;
preferably, the ophthalmic preparations are selected from eye drops, eye ointments, ophthalmic gels, ophthalmic emulsions, ophthalmic suspensions, ophthalmic films, ophthalmic solutions, and intraocular injections; and
preferably, the topical dermatological preparations are selected from aerosols, powders, lotions, tinctures, liniments, films, ointments, gels, pastes, and emulsions.

5. Use of a CYP450 inhibitor in the preparation of drugs for preventing and/or treating xerophthalmia.

6. The use according to claim 5, wherein the xerophthalmia has one or more symptoms selected from eye itching, foreign body sensation, burning sensation, photophobia, blurred vision, fluctuated vision, dry eyes, easy fatigue of eyes, thick secretions, sensitivity to topical stimuli, redness and swelling of eyes, congestion, keratinization, and broken corneal epithelium with filamentous adhesion.

7. The use according to claim 5, wherein the drugs are ophthalmic preparations; preferably, the ophthalmic preparations are selected from eye drops, eye ointments, ophthalmic gels, ophthalmic emulsions, ophthalmic suspensions, ophthalmic films, ophthalmic solutions, and intraocular injections.

8. The use according to any one of claims 1 to 7, wherein the CYP450 is selected from one, two or more of CYP1A1, CYP1A2, CYP1B1, CYP2A6, CYP2B6, CYP2C6, CYP2C8, CYP2C9, CYP2C10, CYP2C19, CYP2D6, CYP2E1, CYP3A3, CYP3A4, CYP3A5, CYP3A7, and Pan CYP;
preferably, the CYP450 inhibitor comprises a substance capable of inhibiting at least one, two or more of CYP1A2, CYP2A6, CYP2B6, CYP2C8, CYP2C19, CYP2D6, and Pan CYP.

9. The use according to any one of claims 1 to 7, wherein the CYP450 inhibitor is selected from dihydroanthraquinone, emodin, 1,4-naphthoquinone, resveratrol, methoxypsoralen, diosmetin, and pharmaceutically acceptable salts, esters, stereoisomers, derivatives, prodrugs, and solvates thereof.

10. The use according to any one of claims 1 to 7, wherein the CYP450 inhibitor is selected from acyclovir, amiodarone, atazanavir, caffeine, cimetidine, ciprofloxacin, enoxacin, famotidine, flutamide, fluvoxamine, lidocaine, lomefloxacin, mexiletine, moclobemide, norfloxacin, ofloxacin, perphenazine, propafenone, ropinirole, tacrine, ticlopidine, tocainide, verapamil, and pharmaceutically acceptable salts, esters, stereoisomers, derivatives, prodrugs, and solvates thereof.

11. The use according to any one of claims 1 to 7, wherein the CYP450 inhibitor is selected from 1,2-phenylenebis(methylene)selenocyanate, 1,3-phenylenebis(methylene)selenocyanate, 1,4-phenylenebis(methylene)selenocyanate, α-naphthoflavone, acetylene, 2-ethynylpyrene, hesperetin, homoeriodictyol, acacetin, diosmetin, resveratrol, oltipraz, 2,4,3',5'-tetramethoxystilbene (TMS), hydroxystyrenes, flutamide, paclitaxel, mitoxantrone, docetaxel, tamoxifen, doxorubicin, daunorubicin, trans-stilbene analogues, imperatorin, isopimpinellin, purpurin, alizarin, polycyclic aromatic hydrocarbons, apigenin, kaempferol, quercetin, amentoflavone, quercetin, rutin, trans-resveratrol methyl ethers, 3',4'-dimethoxyflavone, 5,7,4'-trimethoxyflavone, curcumin, 7,4'-dimethoxyflavone, 7,3'-dimethoxyflavone, thiomethylstilbenes, 2,2',4,6'-tetramethoxystilbene, methoxyflavonoids, melatonin, 2,3,4-trimethoxy-4'-methylthiostilbene, propargyloxyflavone, 4'-methoxy-5,7-dihydroxyflavone, 3'-fluoro-6,7,10-trimethoxy-alpha-naphthoflavone, coumarin, and pharmaceutically acceptable salts, esters, stereoisomers, derivatives, prodrugs, and solvates thereof.

12. The use according to any one of claims 1 to 7, wherein the CYP450 inhibitor is selected from letrozole, clotrimazole, tranylcypromine, pilocarpine, miconazole, amiodarone, ketoconazole, memantine, amphetamine, fenofibrate, methoxsalen, metyrapone, azelastine, clofibrate, fomepizole, isoniazid, menadione, nilvadipine, rosiglitazone, selatrodast, azithromycin, nicotine, triclabendazole, selegiline, and pharmaceutically acceptable salts, esters, stereoisomers, derivatives, prodrugs, and solvates thereof.

13. The use according to any one of claims 1 to 7, wherein the CYP450 inhibitor is selected from ticlopidine, orphenadrine, clotrimazole, itraconazole, raloxifene, methimazole, rilpivirine, memantine, clopidogrel, thiotepa, curcumin, sorafenib, tamoxifen, ketoconazole, crisaborole, manidipine, piperaquine, lopinavir, amprenavir, simvastatin, nelfinavir, selegiline, amlodipine, desipramine, doxorubicin, phencyclidine, azelastine, colchicine, ethanol, isoflurane, miconazole, quinidine, roxithromycin, sulfaphenazole, nitric oxide, cisplatin, regorafenib, enzalutamide, quazepam, crizotinib, enasidenib, voriconazole, safinamide, lenvatinib, rifamycin, triclabendazole, paroxetine, pexidatinib, fluvoxamine, modafinil, curcumin sulfate, abemaciclib, elexacaftor, cenobamate, sertraline, lopinavir, ritonavir, methylene blue, abametapir, cedrol, and pharmaceutically acceptable salts, esters, stereoisomers, derivatives, prodrugs, and solvates thereof.

14. The use according to any one of claims 1 to 7, wherein the CYP450 inhibitor is selected from gemfibrozil, clopidogrel, felodipine, mometasone furoate, zafirlukast, sorafenib, erlotinib, dabrafenib, candesartan cilexetil, salmeterol, trametinib, fluticasone, fluticasone furoate, fluticasone propionate, ritonavir, clotrimazole, ketoconazole, rucaparib, pazopanib, cabozantinib, atazanavir, terbinafine, rofecoxib, quinidine, fenofibrate, bezafibrate, cimetidine, ketoprofen, pyrimethamine, ticlopidine, idelalisib, belinostat, candesartan, opicapone, tegaserod, abiraterone, ubrogepant, amoxicillin, rosiglitazone, trimethoprim, tamoxifen, irbesartan, quinine, efavirenz, rabeprazole, crisaborole, nabilone, bexarotene, nicardipine, loratadine, eltrombopag, diltiazem, enzalutamide, fluvastatin, levothyroxine, oxybutynin, medroxyprogesterone acetate, spironolactone, amlodipine, saquinavir, genistein, lenvatinib, pioglitazone, nilotinib, teriflunomide, topiostat, lovastatin, troglitazone, amitriptyline, cerivastatin, warfarin, lapatinib, raloxifene, quercetin, ethinylestradiol, colchicine, isoniazid, metronidazole, nilutamide, phenelzine, piroxicam, sulfaphenazole, terfenadine, triazolam, valproic acid, diethylstilbestrol, vimodigel, regorafenib, lumacaftor, midostaurin, enasidenib, letermovir, bosutinib, deferasirox, rifampicin, verapamil, simvastatin, sulfinpyrazone, montelukast, nilvadipine, liotrix, mometasone, mifepristone, vemurafenib, licofelone, rutin, ponatinib, isavuconazole, rifamycin, triclabendazole, alpelisib, balaglitazone, ciglitazone, lobeglitazone, netoglitazone, rivoglitazone, tolbutamide, nifedipine, cholecalciferol, atorvastatin, losartan, mefenamic acid, troleandomycin, ezetimibe, anastrozole, abemaciclib, elexacaftor, favipiravir, methylene blue, selpercatinib, riperatinib, clofazimine, saquinavir, miconazole, and pharmaceutically acceptable salts, esters, stereoisomers, derivatives, prodrugs, and solvates thereof.

15. The use according to any one of claims 1 to 7, wherein the CYP450 inhibitor is selected from fluvoxamine, chloramphenicol, delavirdine, gemfibrozil, slipantol, fluoxetine, imipramine, clomipramine, lansoprazole, isoniazid, zafirlukast, tioconazole, miconazole, sertraline, efavirenz, amorphanib, eslicarbazepine acetate, abiraterone, zucasin, ticlopidine, cisapride, manidipine, artemisinol, lopinavir, omeprazole, voriconazole, esomeprazole, pantoprazole, rucaparib, dovitinib, oritavancin, zonisamide, luliconazole, bortezomib, nilutamide, sitaxsentan, clozapine, ethanol, nilvadipine, olanzapine, tipranavir, naloxol, midostaurin, etoricoxib, sildenafil, citalopram, memantine, dexlansoprazole, clinafloxacin, fenofibrate, loratadine, ubugepam, amiodarone, oxazimastat, moclobemide, nicardipine, indomethacin, progesterone, felbamate, rabeprazole, troglitazone, amitriptyline, ritonavir, mephenytoin, tranylcypromine, oxcarbazepine, ketoconazole, thalidomide, aminopyrine, fluvastatin, quinine, warfarin, diazepam, cimetidine, probenecid, carbamazepine, buprenorphine, dimethyl sulfoxide, losartan, phenelzine, sulfonamide, telmisartan, methimazole, valproic acid, sorafenib, bicalutamide, amividipine, vimodigel, idelaris, tropiastat, lobeglitazone, dothiepin, benzbromarone, encidipine, piperaquine phosphate, isaconazole, safinamide, lenvatinib, methsuximide, etravirine, modafinil, azelastine, amprenavir, gefitinib, cerioxetine, rifamycin, fluconazole, triclabendazole, apelis, lynestrenol, ethinylestradiol, gestodene, paroxetine, aprepitant, avasimibe, curcumin sulfate, eslicarbazepine, elexacaftor, topiramate, selenourethane, atorvastatin, cyclosporin, letrozole, methylene blue, and pharmaceutically acceptable salts, esters, stereoisomers, derivatives, prodrugs, and solvates thereof.

16. The use according to any one of claims 1 to 7, wherein the CYP450 inhibitor is selected from thioridazine, paroxetine, cinacalcet, bupropion, levomepromazine, fluoxetine, midostaurin, propafenone, chlorambucil, halofantrine, cisapride, dacomitinib, orphenadrine, quinidine, fluvoxamine, venlafaxine, duloxetine, chlorpromazine, darifenacin, clozapine, celecoxib, cimetidine, tranylcypromine, chloroquine, lumefantrine, nilotinib, cholecalciferol, abiraterone, clobazam, rolapentan, pabistat, rucaparib, manidipine, tilorisan, curcumin, delavirdine, tipranavir, verazodone, phenylpentanoic acid, sulconazole, rhein, asurevir, ritanserin, fusidic acid, lercanidipine, perhexiline, metoprolol, desipramine, clotrimazole, imipramine, quinine, ketoconazole, dothiepin, terfenadine, cyclosporine, sulfaphenazole, tegaserod, ritonavir, terbinafine, primaquine, nicardipine, lorcaserin, milabegron, dronedarone, risperidone, pindolol, loratadine, propranolol, imatinib, lansoprazole, mefloquine, omeprazole, selegiline, methimazole, verapamil, vinblastine, vinorelbine, temsirolimus, rabeprazole, deramciclane, peginterferon alfa -2b, entacapone, ospemifene, buprenorphine, amlodipine, cobistat, ziprasidone, amitriptyline, vemurafenib, reboxetine, nevirapine, fluphenazine, proguanil, nefazodone, dexfenfluramine, etoricoxib, epinastine, lovastatin, trospium chloride, gefitinib, lomustine, st. John's Wort, dexamphetamine, divenlafaxine, amiodarone, clinafloxacin, isoniazid, escitalopram, pazopanib, asenapine, sertraline, ubuazepam, ranolazine, oxizolastat, oritavancin, lidocaine, hydroxyzine, fenfluramine, dextropropoxyphene, tamoxifen, perphenazine, promethazine, chlorpheniramine, acebutolol, moclobemide, miconazole, rotigotine, atorvastatin, cerivastatin, tripelennamine, sparteine, mibefradil, piprazine, biperiden, hydroxyurea, hydroxychloroquine, labetalol, mifepristone, oxprenolol, rosiglitazone, sulfonamide, sorafenib, bicalutamide, dexmedetomidine, indisulfonamide, tapentadol, naloxol, oxymetholone, slipantol, levalbuterol, vernacalan, enciidipine, artemisinol, melperone, isaconazole, black cohosh, phenelzine, safinamide, iproniazid, lenvatinib, rilpivirine, diacerein, rifamycin, dapoxetine, triclabendazole, citalopram, clemastine, clomipramine, cocaine, diphenhydramine, doxepin, doxorubicin, dexchlorpheniramine, mizolastine, trazodone, methadone, metoclopramide, ranitidine, haloperidol, amoxapine, dexchlorpheniramine maleate, ticlopidine, thiothixene, nifedipine, indinavir, oxybutynin, pimozide, azelastine, olanzapine, felodipine, mepyramine, dimethyl sulfoxide, nicotinamide, nicotinic acid, efavirenz, nelfinavir, amodiaquine, oxamniquine, bepridil, 1-(2-phenylethyl)-4-phenyl-4-acetoxypiperidine, cannabidiol, medical marijuana, nabiximols, curcumin sulfate, eliglukast, everolimus, flecainide, ampicillin, lamiditan, elexacaftor, nortriptyline, darunavir, fizzotinib, methylene blue, clofazimine, and pharmaceutically acceptable salts, esters, stereoisomers, derivatives, prodrugs, and solvates thereof.

17. The use according to any one of claims 1 to 7, wherein the CYP450 inhibitor is selected from clomethiazole, disulfiram, diethyl dithiocarbamate, isothiocyanic acid, S-adenosylmethionine, insulin, and pharmaceutically acceptable salts, esters, stereoisomers, derivatives, prodrugs, and solvates thereof.

18. The use according to any one of claims 1 to 7, wherein the CYP450 inhibitor is selected from telithromycin, clarithromycin, itraconazole, ketoconazole, indinavir, ritonavir, saquinavir, nefazodone, diltiazem, erythromycin, fluconazole, verapamil, cimetidine, amiodarone, amprenavir, aprepitant, ciprofloxacin, doxycycline, enoxacin, fluvoxamine, imatinib, miconazole, voriconazole, cedrol, and pharmaceutically acceptable salts, esters, stereoisomers, derivatives, prodrugs, and solvates thereof.

19. The use according to any one of claims 1 to 7, wherein the CYP450 inhibitor is selected from atipamezole, 1-aminobenzotriazole, prodifene, acetylshikonin, ketoconazole, chlorophyllin, cimetidine, satraplatin, metyrapone, miconazole, 17-octadecanoic acid, stripentol, amiodarone, and pharmaceutically acceptable salts, esters, stereoisomers, derivatives, prodrugs, and solvates thereof.

20. The use according to any one of claims 1 to 7, wherein the CYP450 inhibitor is selected from quinidine, montelukast, quercetin, sulfaphenazole, methoxsalen, ketoconazole, itraconazole, tranylcypromine, alpha-naphthoflavone, atipamezole, sertraline, cimetidine, curcumin, thiotepa, permethrin, clotrimazole, miconazole, paroxetine, cisapride, gemfibrozil, clopidogrel, zafirlukast, sorafenib, ritonavir, voriconazole, triclabendazole, orphenadrine, felodipine, memantine, letrozole, fluoxetine, midostaurin, metronidazole, cedrol, β-cedrene, bisabolol, chamazulene, pogostone, and pharmaceutically acceptable salts, esters, stereoisomers, derivatives, prodrugs, and solvates thereof;
preferably, the CYP450 inhibitor is selected from quinidine, montelukast, quercetin, tranylcypromine, sertraline, thiotepa, clotrimazole, paroxetine, orphenadrine, methoxsalen, clopidogrel, memantine, cimetidine, curcumin, midostaurin, metronidazole, cedrol, β-cedrene, bisabolol, chamazulene, pogostone and pharmaceutically acceptable salts, esters, stereoisomers, derivatives, prodrugs, and solvates thereof; and
more preferably, the CYP450 inhibitor is selected from quinidine, montelukast, quercetin, tranylcypromine, sertraline, thiotepa, clotrimazole, paroxetine, orphenadrine, cedrol, bisabolol, chamazulene, pogostone, and pharmaceutically acceptable salts, esters, stereoisomers, derivatives, prodrugs, and solvates thereof.
